# FASCICULE DE BREVET EUROPEEN

(11) **EP 0 562 927 B1**
(45) Date de publication et mention de la délivrance du brevet: **03.07.1996**
(21) Numéro de dépôt: 93400713.9
(22) Date de dépôt: 19.03.1993
(51) Int. Cl.: C08B 31/18, C08B 31/12

(54) **Procédé d'oxydation d'amidons cationiques et amidons amphotères carboxyliques et cationiques ainsi obtenus**
Verfahren zur Oxidation von kationischer Stärke und damit erhältliche, amphotere, kationische Carboxylstärke
Process for oxidising cationic starch and amphoteric cationic carboxylic starch obtained thereby

(30) Priorité: 23.03.1992 FR 9203465
(43) Date de publication de la demande: 29.09.1993
(73) Titulaire: ELF ATOCHEM S.A., F-92800 Puteaux (FR)
(72) Inventeur: Suc, Sophie, F-69600 Oullins (FR); Defaye, Jacques, F-38330 Saint Ismier (FR); Gadelle, Andrée, F-38330 Montbonnot Saint Martin (FR); Kervennal, Jacques, F-69005 Lyon (FR)
(74) Mandataire: Kaplan, Jean-Pierre

(56) Documents cités:
- US-A- 3 706 584
- CHEMICAL ABSTRACTS, vol. 116, no. 11, 1991, Columbus, Ohio, US; abstract no. 105703y, & IND. CHEM. LIBR. vol. 3, no. 1, 1991, pages 127-134 REEVE K. M. ET AL. "Oxidation with hydrogen peroxide and hydrogen bromide"

## Description

La présente invention concerne un procédé d'oxydation d'amidons cationiques comportant des groupes terminaux réducteurs.

L'invention a également pour objet les nouveaux amidons amphotères, comportant à la fois des groupements carboxyliques et cationiques, susceptibles d'être obtenus par ce procédé.

Il est connu de modifier chimiquement l'amidon. Ainsi, l'ouvrage "Modified Starches : properties and uses" de O.B. Wurzburg, publié par C.R.C. Press, Inc, en 1986, fait état de procédés d'oxydation de l'amidon par le brome, le chlore ou encore les hypohalogénites alcalins correspondants. Ces réactifs oxydent l'amidon de manière non sélective, et le plus généralement, on admet que :
- les groupes hydroxyles primaires en position C-6 peuvent être oxydés en groupes carboxyliques conduisant à des motifs acide uronique;
- les groupes hydroxyles secondaires en position C-2, C-3, C-4 sont susceptibles d'être oxydés en cétones ;
- les groupes α-glycols peuvent être oxydés en dialdéhydes par rupture oxydante de la liaison C-C, puis en dérivés dicarboxyliques.
- les groupes hémiacétaliques de l'amylose et de l'amylopectine sont oxydés en groupes carboxyliques ou en lactones.

Ainsi, I.I. Kuznetsova et N.N. Tregubov, Sakh. Promst.,1984, 9, 47-50 ont fait réagir un amidon de maïs avec du peroxyde d'hydrogène (0,1% en poids par rapport à l'amidon) et de l'acide chlorhydrique (0,15% en poids par rapport à l'amidon) à des températures comprises entre 140 et 180 °C, par chauffage IR pendant une durée comprise entre 2 et 10 minutes.

D'après ces auteurs, la quantité de groupes carboxyliques formés est comprise entre 0,003 et 0,006% à 140 °C, 0,005 et 0,020% à 160 °C, et 0,007 et 0,050% à 180 °C. Cette oxydation n'est pas sélective car elle entraîne la formation de substances réductrices (S.R.) dont la teneur varie entre 1 et 10% .

Par ailleurs, L.J. Torneport et Coll., Die Stärke,1990, 42, 413-417, décrivent l'oxydation par le brome en solution aqueuse à pH 7, d'une suspension d'amidon de pomme de terre. Ce procédé provoque également la formation simultanée de groupes carbonylés et carboxyliques.

A.C.B. Salomonson et coll., Carbohydr. Res.,1991, 217, 221-225, ont déterminé, par RMN ¹H et ¹³C, la composition du mélange obtenu dans le procédé précité. Ces auteurs ont confirmé l'introduction de groupes carbonyles en C-2 et C-3 des motifs anhydroglucose et la formation d'acides carboxyliques sans noter toutefois la présence d'acides aldoniques résultant de l'oxydation du groupe réducteur terminal en acide carboxylique.

L'amidon peut faire l'objet d'autres types de modifications chimiques et, comme le rappellent R.L. Whistler et coll., dans Kirk-Othmer edit. "Encyclopedia of Chemical Technology,Starch,1983,21, pp 492-507", l'amidon en tant que structure polyhydroxylée peut subir les réactions caractéristiques des alcools et notamment l'estérification et l'éthérification.

Les dérivés ainsi modifiés de l'amidon, les plus importants commercialement, ont en général un degré de substitution (DS) inférieur à 0,1. Ce DS, même faible, produit des changements importants dans les propriétés physico-chimiques de ces polymères.

Parmi ces dérivés de substitution, on connaît en particulier les amidons cationiques.

Les amidons cationiques sont définis comme étant des amidons dans lesquels certains hydroxyles ont été éthérifiés par des groupements cationiques ou susceptibles d'être transformés en milieu acide en groupes cationiques par protonation. Par exemple, ces substituants peuvent comporter des fonctions amine tertiaire ou sels d'ammonium quaternaire, ou encore des groupes sulfonium ou phosphonium.

Le brevet américain US 3 654 263 (C.P.C. International Inc.) décrit les méthodes générales d'obtention d'amidons cationiques et son enseignement est incorporé dans la présente demande. De préférence, les amidons cationiques servant de produit de départ dans la présente demande ont un DS cationique ou DS_{c} compris entre environ 0,01 et 0,1.

Certains amidons cationiques sont commerciaux et utilisés industriellement, notamment dans la composition de revêtement pour la fabrication du papier, car ils augmentent la rétention des pigments et éléments fins, ainsi que la résistance mécanique (rupture, éclatement, déchirure, cohésion interne).

Parmi les amidons modifiés chimiquement, on connaît également les amidons carboxyliques et cationiques qui se définissent comme étant des amidons amphotères comportant à la fois des groupes carboxyliques (-CO₂H) et des groupes cationiques. Les groupes carboxyliques peuvent être formés à partir d'atomes de carbone du motif structural anhydroglucose par oxydation.

Ainsi, les brevets américains US 3 598 623 et 3 649 624 (A.E. Staley, Manufacturing Company) décrivent des amidons carboxyliques et cationiques, obtenus notamment par oxydation d'amidons cationiques de type amino-éther tertiaire ou sel d'ammonium quaternaire.

La préparation des amidons carboxyliques comportant des substituants éther-amine tertiaire requiert, dans certains cas, que l'amidon soit tout d'abord oxydé pour donner un amidon carboxylique, puis que ce dernier soit éthérifié par un groupement porteur d'une fonction amine tertiaire. En effet, les groupes amines tertiaires sont relativement instables dans les conditions d'oxydation utilisées pour conduire à un amidon carboxylique.

Les exemples I à XI de ces brevets Staley décrivent l'obtention d'amidons amphotères carboxyliques-sels d'ammonium quaternaire où l'agent d'alkylation est le chlorure de 3-chloro-2-hydroxypropyltriméthylammonium et l'oxydant, l'hypochlorite de sodium.

Le degré de substitution de ces amidons par les groupements carboxyliques est dénommé degré de substitution anionique (Dsₐ).

Le degré de substitution par les radicaux ammonium tertiaire est dénommé degré de substitution cationique (Ds_{c}).

Le Tableau I résume les caractéristiques des amidons modifiés obtenus dans les exemples I à XI.

**TABLEAU I**

| Amidon + (-CO₂⁻, >N<) | DSₐ (-CO₂H) | DS_{c} + (>N<) | DSₐ/DS_{c} |
|---|---|---|---|
| A | 0,029 | 0,016 | 1,8 |
| B | 0,050 | 0,016 | 3,1 |
| C | 0,011 | 0,0148 | 0,74 |
| D | 0,029 | 0,028 | 1,03 |
| E | 0,032 | 0,029 | 1,1 |
| F | 0,050 | 0,029 | 1,7 |
| G | 0,050 | 0,036 | 1,4 |
| H | 0,050 | 0,040 | 1,25 |
| I | 0,038 | 0,029 | 1,3 |
| J | 0,022 | 0,018 | 1,2 |
| K | 0,037 | 0,029 | 1,3 |
| L | 0,026 | 0,029 | 0,89 |
| M | 0,041 | 0,017 | 2,4 |
| N | 0,041 | 0,022 | 1,8 |

Les amidons C et L, dont le rapport Dsₐ/Ds_{c} est inférieur à 1, produiraient avec les pigments utilisés un effet dit de "choc" conduisant à des mélanges non homogènes,extrêmement visqueux, et par conséquent ne conviendraient pas pour des applications papetières.

Les brevets américains US 3 654 263 et 3 706 584 (C.P.C. International Inc.) décrivent l'oxydation d'amidons cationiques par action de l'hypochlorite de sodium. Cette oxydation introduit à la fois des fonctions carbonyle et carboxyle dans les molécules d'amidon cationique tout en réduisant leur taille moyenne, à savoir leur degré de polymérisation (D.P.).

Les exemples 1 et 2 décrivent l'alkylation d'amidons par le chlorure de 3-chloro-2-hydroxylpropyltriméthylammonium, obtenu par réaction de la triméthylamine et de l'épichlorhydrine.

Les analyses, formulées en pourcentages en poids de groupes carboxyliques, permettent de calculer le DSₐ de la même manière que dans les brevets Staley ci-dessus.

Les caractéristiques des amidons amphotères obtenus , carboxylique-sels d'ammonium quaternaire, sont rassemblées dans le Tableau II.

**TABLEAU II**

| Amidon (-CO₂⁻, >N⁺<) | % en poids groupes -CO₂⁻ | DSₐ | DS_{c} | DSₐ/DS_{c} |
|---|---|---|---|---|
| 1A | 0,10 | 0,0037 | 0,025 | 0,148 |
| 1B | 0,30 | 0,011 | 0,021 | 0,52 |
| 1C | 0,28 | 0,010 | 0,015 | 0,66 |
| 1D | 0,26 | 0,0096 | 0,019 | 0,50 |
| 2A | 0,18 | 0,0066 | 0,014 | 0,47 |
| 2B | 0,31 | 0,0114 | 0,014 | 0,81 |
| 2C | 0,51 | 0,018 | 0,014 | 1,28 |
| 2D | 0,09 | 0,003 | 0,019 | 0,157 |
| 2E | 0,16 | 0,006 | 0,024 | 0,25 |

Les amidons amphotères ainsi obtenus entrent dans la composition de revêtements pour papier.

Le but principal de la présente invention est de définir une nouvelle classe d'amidons amphotères utilisables en particulier en papeterie.

Un autre but est de mettre en oeuvre un procédé d'oxydation sélective des groupes réducteurs terminaux d'un amidon cationique et éventuellement de ses produits d'hydrolyse acide, permettant d'obtenir ces amidons amphotères.

D'une manière surprenante, si l'on considère l'état de la technique rapportée ci-dessus concernant l'oxydation de l'amidon par le brome, les objectifs précités sont atteints, selon la présente invention, par un procédé d'oxydation d'un amidon cationique comportant des groupes terminaux réducteurs, caractérisé en ce qu'on fait réagir, en solution aqueuse, ledit amidon cationique avec du peroxyde d'hydrogène et un composé halogéné choisi parmi l'acide bromhydrique et le brome, de manière à oxyder sélectivement les groupes réducteurs terminaux de l'amidon cationique et éventuellement les groupes réducteurs de ses produits d'hydrolyse acide, en groupes carboxyliques.

Le terme "groupe carboxylique" signifie groupe acide carboxylique (-CO₂H) ou encore la lactone correspondante, ces deux structures pouvant être en équilibre selon le pH auquel est exposé l'amidon carboxylique-cationique obtenu.

Par traitement de la solution aqueuse après l'oxydation, notamment par ajout d'un alcool, on isole un amidon carboxylique et cationique sous forme d'un précipité solide.

Avantageusement, l'alcool est choisi parmi le méthanol et l'éthanol. Le solide qui précipite est recueilli par filtration. L'amidon amphotère carboxylique et cationique obtenable par le procédé selon l'invention se caractérise en ce que ses groupes carboxyliques proviennent de l'oxydation sélective des groupes hémiacétaliques terminaux d'un amidon cationique de départ et éventuellement des groupes hémiacétaliques terminaux de ses produits d'hydrolyse acide.

En effet, il est connu que le traitement des amidons naturels par des acides en présence d'eau, conduit à l'hydrolyse des liaisons interosidiques α-(1 --> 4) et α-(1--> 6) dans les molécules d'amidons naturels (voir Supra dans Starch,1983,21, pp 492-507). Cette hydrolyse a pour effet d'augmenter le nombre des groupes terminaux réducteurs dans les produits d'hydrolyse acide des amidons.

Les amidons cationiques gardent, au moins en partie, la structure de base polymérique anhydroglucose de l'amylose et de l'amylopectine de l'amidon et en particulier la susceptibilité à l'hydrolyse acide des liaisons α-(1 --> 4) et α-(1 --> 6).

Les amidons cationiques sont définis comme dans l'introduction ci-dessus de la présente demande.

Les amidons cationiques utilisables comme matière première dans le procédé selon l'invention doivent être solubles ou solubilisables dans l'eau, afin que l'oxydation selon l'invention soit à la fois sélective et suffisamment rapide pour une exploitation industrielle.

Avantageusement, le procédé est appliqué à un amidon cationique dans lequel les substituants cationiques sont constitués par des groupements sels d'ammonium quaternaire.

Ces groupes ammonium quaternaire sont de préférence greffés sur les molécules d'amidons naturels par éthérification des groupes hydroxyle libres par des agents alkylant comportant un groupe amine tertiaire ou un sel d'ammonium quaternaire.

Les groupes aminés tertiaires, après éthérification de l'amidon, peuvent être quaternarisés de manière classique par des produits alkylants tels l'iodure ou le bromure de méthyle ou le sulfate d'éthyle ou de méthyle.

De préférence, les agents alkylants sont choisis parmi le chlorure de 2,3-époxypropyltriméthylammonium, le chlorure de 2-diéthylaminoéthyle, leurs analogues arylamines et amines hétérocycliques, les halohydrates d'amines tertiaires, telles que le 3-dibutylamino-1,2-époxypropane, la N-(2,3-époxypropyl) pipéridine, la N-(2,3-époxypropyl)N-méthylaniline, le bromhydrate de 2-bromo-5-diéthylaminopentane, le chlorure de β-diéthylaminoéthyle, le chlorure de β-diméthylaminoéthyle, ou encore les produits de réaction de l'épichlorhydrine ou de l'épibromhydrine avec des amines tertiaires ou halohydrates d'amines tertiaires, telles que la triméthylamine, la triéthylamine, le chlorhydrate de triméthylamine, le bromhydrate de triéthylamine, la N,N-diméthyldodécylamine, la N,N-diméthylcyclohexylamine, la N,N-diméthylaniline, la N,N-diméthylbenzylamine, la N-méthylmorpholine, la N-méthylpiperidine, et la N-méthylpyrrolidine.

Les amidons utilisés pour l'obtention d'amidons cationiques peuvent être d'origine quelconque, dans la mesure où ils comportent des groupes hydroxyle libres pouvant être estérifiés ou éthérifiés.

Ces amidons peuvent être avantageusement choisis parmi les amidons non modifiés chimiquement, issus d'amidons de blé, de maïs, et en particulier ceux enrichis en amylopectine, de riz, de pommes de terre, de tapioca, de marante, de sorgho. Ces amidons peuvent être également choisis parmi les amidons modifiés par hydrolyse partielle acide, enzymatique ou thermique des amidons naturels.

Avantageusement, le procédé d'oxydation selon l'invention est conduit de façon à ce que ladite solution aqueuse réactionnelle soit maintenue lors de l'oxydation à un pH inférieur à 7, ou de manière toute préférée entre 4 et 6.

De préférence, la solution aqueuse réactionnelle est maintenue à une température comprise entre 20 et 60 °C.

De préférence, le peroxyde d'hydrogène (H₂O₂) est en quantité molaire supérieure à la quantité molaire du composé halogéné HBr ou Br₂.

Le rapport molaire H₂O₂/Br₂ est avantageusement compris entre 1 et 200.

En présence d'un grand excès de peroxyde d'hydrogène par rapport au brome ou à l'acide bromhydrique,ces derniers se comportent comme des moyens équivalents dans la mesure où il est connu de W.C. Bray et coll., J. Am. Chem. Soc., 1928, 50, 1654, que H₂O₂ oxyde en solution aqueuse HBr en Br₂ selon l'équation : H₂O₂ + 2HBr -----> Br₂ + 2H₂O

Dans le procédé selon l'invention tout composé bromé pouvant donner du brome sous l'action de peroxyde d'hydrogène, pourrait remplacer l'ajout de HBr ou de Br₂ dans la solution réactionnelle.

Le rapport molaire H₂O₂/HBr est avantageusement compris entre 0,5 et 100.

Un des avantages du procédé selon l'invention réside dans le fait que le brome en faible teneur oxyde sélectivement les groupes hémiacétal cycliques selon la réaction : 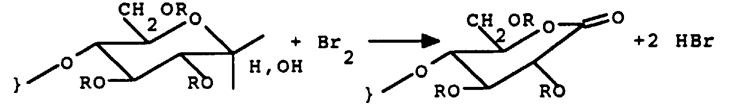 R étant statistiquement un hydrogène ou, avec une faible probabilité pour les faibles DS_{c}, un substituant cationique.

L'acide bromhydrique ainsi produit est réoxydé au fur et à mesure de sa formation par le peroxyde d'hydrogène présent en solution.

La faible teneur en HBr permet de limiter considérablement l'hydrolyse des liaisons glycosidiques α-(1 --> 4) et/ou α-(1 --> 6) des polymères du type anhydroglucose.

Avantageusement, le rapport molaire du peroxyde d'hydrogène sur les unités anhydroglucose de l'amidon cationique de départ, est compris entre 0,1 et 10.

La présente invention sera mieux comprise à l'aide des exemples qui vont suivre.

Les exemples de réalisation du procédé revendiqué sont donnés à titre purement illustratif, alors que les exemples du même procédé appliqué à des composés modèles ont pour but d'étayer la sélectivité de l'oxydation des groupes hémiacétal cyclique en présence de groupes hydroxyle dans des structures glucose ou anhydroglucose de faible degré de polymérisation (DP).

Dans ces exemples, la viscosité est mesurée à l'aide d'un appareil AVS 400 de la Société Schott-Gerät muni d'un tube de UBBELOHDE dans le diméthylsulfoxyde (DMSO).

L'amidon STA-LOK 180 ^{R}, de la Société américaine A.E. STALEY a les caractéristiques suivantes :

| | |
|---|---|
| Teneur en amylose | ≤ 5 % |
| Teneur en amylopectine | ≥ 95 % |
| Nature cationique | sel d'ammonium quaternaire avec une liaison éther |
| % en poids d'azote | 0,35 |
| Degré de substitution cationique, DS_{c} | 0,03 |
| Viscosité pour 3% de solide dans l'eau à 66 °C | 286 mPa.s |
| Viscosité déterminée en tube de UBBELOHDE (type 537), pour une solution à 0,5% en poids dans le DMSO à 25°C | η = 40,91 mm²/s |
| Teneur en halogénure | < 250 p.p.m. |

Cet amidon se solubilise à plus de 98 g pour 100 g d'eau par chauffage à 98 °C puis refroidissement.

L'amidon HI-CAT 180 de la Société ROQUETTE FRERES a les caractéristiques suivantes :

| | |
|---|---|
| % en poids d'azote | 0,6 |
| Nature cationique | sel d'ammonium quaternaire avec liaison éther |
| Degré de polymérisation moyen | non connu |

Cet amidon se solubilise dans l'eau à température ambiante.
Viscosité UBBELOHDE (type 537)
à 0,25% en poids dans le DMSO : η = 2,8 mm²/s

Le taux d'acidité, ou taux de groupements carboxyliques ou taux d'oxydation est défini en pourcentage. Il correspond au nombre de groupements carboxyliques pour 100 unités anhydroglucose de l'amidon modifié. Le degré de substitution est défini comme étant le nombre de groupements carboxyliques pour 1 unité anhydroglucose de l'amidon modifié.

Le peroxyde d'hydrogène et l'acide bromhydrique sont introduits sous forme de solution aqueuse dont le titre (par exemple 30%) exprime la masse en grammes de H₂O₂ ou HBr pour 100 grammes de solution.

### EXEMPLE 1

### Oxydation de l'amidon STA-LOK 180^{R} et amidon amphotère carboxylique-sel d'ammonium quaternaire ainsi obtenu.

Dans un réacteur muni d'un réfrigérant et équipé d'une agitation mécanique, on dissout cet amidon (5 g) dans une solution aqueuse de peroxyde d'hydrogène (30%, 5 ml, 49 mmol, soit 1,6 moles d'H₂O₂ par motif anhydroglucose de cet amidon), à la température ambiante de 20 °C. A la solution visqueuse ainsi obtenue, de l'acide bromhydrique (solution aqueuse à 40% , 0,5 ml, 3,4 mmol) est ajouté en une fois, tout en poursuivant l'agitation. Après 5 heures, la solution visqueuse légèrement jaune est additionnée de méthanol (250 ml). Le précipité obtenu est séparé par filtration et séché jusqu'à poids constant (5 g, 100%). Un titrage potentiométrique, réalisé par addition d'une solution d'hydroxyde de sodium (0,0468 N), en prenant en compte le point d'inflexion de la courbe de neutralisation, permet de déterminer un taux d'acidité de 0,35% correspondant à un taux de groupements carboxyliques ou taux d'oxydation, de 0,15%, soit un degré de substitution DSₐ de 0,0015, si l'on tient compte de la présence résiduelle de 0,20% d'équivalent de brome ionique Br⁻, telle que déterminée par conductimétrie. La proportion de brome total résiduel dans l'échantillon, déterminée par argentométrie est de 0,46% en poids.

La viscosité du produit final (5 g) déterminée en tube de UBBELOHDE (type 537) est de : η = 7,34 mm²/s pour une solution à 0,5% en poids.

Une microanalyse donne une teneur en poids de 0,36 % d'azote, ce qui correspond à un DS_{c} de 0,03.

Le rapport DSₐ/DS_{c} est égal à 0,05

### EXEMPLE 2

Dans des conditions opératoires analogues à celles de l'exemple précédent, on dissout 100 g du même amidon ammonium quaternaire dans un mélange d'eau (100 ml) et d'une solution de peroxyde d'hydrogène (30%, 15 ml, 147 mmol, correspondant à 0,24 mole d'oxydant par motif anhydroglucose de l'amidon), à la température ambiante de 20°C. A la solution visqueuse ainsi obtenue, de l'acide bromhydrique (solution aqueuse à 40% , 1,5 ml, 10,2 mmol) est ajouté en une fois tout en poursuivant l'agitation. Après 5 heures, la solution visqueuse est concentrée sous vide à 30°C pendant 0,5 heure. Le résidu obtenu est additionné de méthanol (500 ml). Le précipité obtenu est séparé par filtration et séché jusqu'à poids constant (100 g, 100 %). Un titrage potentiométrique, réalisé par addition d'une solution d'hydroxyde de sodium (0,0468 N) permet de déterminer un taux d'acidité global de 0,12 - 0,15%, soit un taux d'oxydation de 0,12 - 0,15%, la teneur globale en brome étant de 800 p.p.m.
- Viscosité :: η = 12,6 mm²/s pour une solution à 0,5% dans le DMSO
- DSₐ: = 0,0012 - 0,0015
- Microanalyse :: 0,37 % en poids d'azote, ce qui correspond à un DS_{c} de 0,03
- DSₐ/DS_{c}: = 0,04 - 0,05

### EXEMPLE 3

### Oxydation de l'amidon HI-CAT 180 (Société Roquette Frères).

Dans un appareillage analogue à celui de l'Exemple 1, on dissout cet amidon (20 g) dans un mélange d'eau (20 ml) et d'une solution de peroxyde d'hydrogène (30%, 3 ml, 29,4 mmol, correspondant à 1,24 moles d'oxydant par motif anhydroglucose de cet amidon), à la température ambiante de 20 °C. A la solution très visqueuse obtenue, de l'acide bromhydrique (solution à 40% , 0,3 ml, 2,04 mmol) est ajouté en une fois tout en poursuivant l'agitation. Après 5 heures, la solution très visqueuse est évaporée sous vide pendant 0,5 heure, puis le résidu ainsi obtenu est ensuite additionné de méthanol (500 ml). Le précipité obtenu est séparé par filtration et séché à poids constant (19 g, 95%). Un titrage potentiométrique par de la soude (0,0468 N) permet de déterminer un taux d'acidité de 1.6 10⁻³ correspondant à un taux de groupe carboxylique de 1.6 10⁻³, soit un DSₐ de 1.6 10⁻³ si l'on tient compte de la présence résiduelle de 800 ppm de brome.
- Viscosité :: η = 3,73 mm²/s pour une solution à 0,25% en poids dans le DMSO.

Afin d'étayer le fait que le procédé selon la présente invention oxyde sélectivement les groupes hémiacétals des amidons cationiques ou de leurs produits d'hydrolyse acide sans affecter les groupes hydroxyles primaires ou secondaires des motifs anhydroglucose, les mêmes conditions opératoires d'oxydation ont été appliquées à des composés modèles ayant une structure glucose ou formés de motifs anhydroglucose de faible degré de polymérisation.

### EXEMPLE 4

### Oxydation du D-glucose en acide D-gluconique

Dans une enceinte fermée (flacon à vis de 500 ml), du D-glucose (10 g, 55 mmol) est dissous dans une solution aqueuse de peroxyde d'hydrogène (30% , 5 ml, 49 mmol) avec une agitation magnétique et à température ambiante de 20 °C. De l'acide bromhydrique (solution aqueuse à 40% , 2 ml, 13,6 mmol) est ajouté en une fois, tout en poursuivant l'agitation. Après 24 heures, une solution aqueuse de peroxyde d'hydrogène est ajoutée (70%, 2,5 ml, 68 mmol) et la réaction est poursuivie encore 24 heures. Puis la solution obtenue est additionnée d'eau (25 ml) et débarrassée du brome résiduel par barbotage d'air, avant d'être additionnée d'hydroxyde de calcium (2 g), chauffée et concentrée sous pression réduite jusqu'à un volume d'environ 20 ml. Le gluconate de calcium, qui cristallise spontanément de la solution au bout de quelques heures, est isolé par filtration et recristallisé dans l'eau. On obtient 11 g (92%) d'un produit ayant les caractéristiques suivantes :
[α]D²⁰° = + 8,5° (c = 3, eau)
RMN ¹³c (D₂O, pH = 7, (CH₃)₂CO,δ = 31,07)
178,7 (C-1) ; 74,4 ; 72,6 , 71,3 , 70, 9 (C-2 à C-5) ; 62,7 (C-6).

A titre de comparaison, la littérature donne pour l'acide D-gluconique les valeurs suivantes :
- [α]D^{20°}: = + 8,5°Hudson and Isbell,
J. Am. chem. Soc,1929,51, 2225
- RMN ¹³c: :(pH 14) 179,8 (c-1) ; 75,2 ; 73,8 ; 72, 4 ;
72,0 (c-2 à c-5) ; 63,6 (c - 6) Isbell, carbohydr.
Res., 1981,90, 123.

### EXEMPLE 5

### Oxydation du maltose en acide maltobionique isolé sous la forme de son sel de brucine

Les précautions d'usage sont prises quant à la toxicité de la brucine.

Dans une enceinte fermée (100 ml) contenant du maltose (5 g, 13,9 mmol) dissous dans une solution aqueuse de peroxyde d'hydrogène (70% , 2,5 ml, 68 mmol) est ajouté de l'acide bromhydrique (solution aqueuse à 40% , 0,25 ml, 0,17 mmol). La solution, protégée de la lumière, est laissée sous agitation. Après 24 heures, sont ajoutés une solution aqueuse de H₂O₂ (70%, 2,5 ml, 68 mmol) et de l'acide bromhydrique (solution aqueuse à 40% en poids, 0,25 ml, 0,17 mmol).

Après 48 heures, un spectre de R.M.N. ¹³C, réalisé sur une aliquote du mélange réactionnel, permet de déterminer qu'au moins 85% du maltose de départ a été transformé en acide maltobionique (comparaison des intensités des pics 99,8 (C-1) pour le maltose et 100,2 et 100,6 (C-1) pour le produit d'oxydation). Le brome résiduel est éliminé par barbotage d'air, puis la solution est additionnée d'eau (20 ml) et de brucine (6 g, 13,9 mmol). Les cristaux de sel de brucine sont filtrés (8,2 g, 75%), P.F. = 151 °C. [α]_{D} = +33° (c=1,3 ; H₂O)

A titre de comparaison, la littérature (Carbohydrate, P.M. Collins edit., Chapman 1987) donne les caractéristiques suivantes pour le maltobionate de brucine:
P.F. = 153 °C
[α]_{D}^{20°} = + 38,1° (H₂O)

Cet exemple montre que s'il y a hydrolyse acide de la liaison glucosidique, cette hydrolyse est faible, d'après la R.M.N ¹³C

### EXEMPLE 6

### Oxydation de maltodextrines en acides polyhydroxycarboxyliques

Les maltodextrines utilisées ont un degré de polymérisation moyen (DPₘ) de 5 et proviennent de l'hydrolyse d'un amidon.Elles sont commercialisées sous l'appelation "Glucidex 19" par la Société Roquette Frères (France).

Dans une enceinte fermée (100 ml) contenant une solution de peroxyde d'hydrogène (70% , 2,5 ml, 68 mmol) sont ajoutés des maltodextrines (5 g, 31 mmol d'anhydroglucose correspondant à 5,8 m.équiv. de dextrose) et de l'acide bromhydrique (solution aqueuse à 40%,0,25 ml, 0,17 mmol). La solution fortement visqueuse est agitée magnétiquement à l'abri de la lumière pendant 96 heures à 20 °C. Une aliquote est alors prélevée et analysée en R.M.N. du ¹³C comparativement au produit de départ. On note l'évolution de la réaction d'oxydation comme à l'exemple 5, ci-dessus.

La solution réactionnelle est alors additionnée d'eau (25 ml) et neutralisée par de la soude (0,0468 N, 172 ml). Le produit lyophilisé (5,6 g) est lavé par du méthanol, filtré, redissous dans de l'eau ( 50 ml ) et lyophilisé (5,2 g, 100%). Une microanalyse indique qu'il subsiste 800 ppm de brome résiduel.

En conclusion, les exemples 4, 5 et 6 montrent bien que la réaction d'oxydation revendiquée est sélective des seuls groupes réducteurs hémiacétaliques et que les groupes hydroxyles primaires ou secondaires présents sur les structures considérées ne sont pas réactifs sous ces conditions.

Cette sélectivité repose vraisemblablement sur la faible concentration en brome dans les milieux réactionnels générés selon le procédé de la présente invention et sur le fait que le potentiel oxydant du brome est adapté à l'oxydation sélective de la fonction hémiacétalique qui, par nature, est en équilibre avec la structure hydroxy-aldéhydique correspondante.

## Revendications

1. Procédé d'oxydation d'un amidon cationique comportant des groupes terminaux réducteurs, caractérisé en ce qu'on fait réagir, en solution aqueuse, ledit amidon cationique avec du peroxyde d'hydrogène et un composé halogéné choisi parmi l'acide bromhydrique et le brome, de manière à oxyder sélectivement les groupes réducteurs terminaux de l'amidon cationique et éventuellement les groupes réducteurs de ses produits d'hydrolyse acide, en groupes carboxyliques.

2. Procédé suivant la revendication 1, caractérisé en ce que l'amidon cationique a des substituants cationiques constitués par des groupements sels d'ammonium quaternaire.

3. Procédé suivant la revendication 1 ou 2, caractérisé en ce que l'on maintien ladite solution aqueuse de l'amidon cationique, du peroxyde d'hydrogène et du composé halogéné à un pH inférieur à 7.

4. Procédé suivant la revendication 3, caractérisé en ce que ledit pH est maintenu entre 4 et 6.

5. Procédé suivant l'une des revendications 1 à 4, caractérisé en ce que la solution aqueuse est maintenue à une température comprise entre 20 et 60 °C.

6. Procédé suivant l'une des revendications 1 à 5, caractérisé en ce que le rapport molaire peroxyde d'hydrogène/brome est compris entre 1 et 200.

7. Procédé suivant l'une des revendications 1 à 5, caractérisé en ce que le rapport molaire peroxyde d'hydrogène/acide bromhydrique est compris entre 0,5 et 100.

8. Procédé suivant l'une des revendications 1 à 7, caractérisé en ce que le rapport molaire du peroxyde d'hydrogène sur les unités anhydroglucose de l'amidon cationique de départ, est compris entre 0,1 et 10.

9. Procédé suivant l'une des revendications 1 à 8, caractérisé en ce qu'après l'oxydation de l'amidon cationique, on traite la solution aqueuse de façon à isoler l'amidon carboxylique et cationique sous forme d'un précipité solide.

10. Procédé suivant la revendication 9, caractérisé en ce que l'on ajoute à la solution aqueuse un alcool pour précipiter l'amidon carboxylique et cationique.

11. Procédé suivant la revendication 10, caractérisé en ce que l'alcool est choisi parmi le méthanol et l'éthanol.

12. Amidon amphotère carboxylique et cationique, caractérisé en ce que ses groupes carboxyliques proviennent de l'oxydation sélective des groupes hémiacétaliques terminaux d'un amidon cationique et éventuellement des groupes hémiacétaliques terminaux des produits d'hydrolyse acide de cet amidon cationique.

## Patentansprüche

1. Verfahren zur Oxidation von kationischer Stärke mit reduzierenden Endgruppen,
dadurch gekennzeichnet, daß
die kationische Stärke in wäßriger Lösung mit Wasserstoffperoxid und einer Halogenverbindung, die unter Bromwasserstoffsäure und Brom ausgewählt ist, so umgesetzt wird, daß die reduzierenden Endgruppen der kationischen Stärke und ggf. die reduzierenden Gruppen ihrer durch saure Hydrolyse gebildeten Produkte selektiv zu Carboxygruppen oxidiert werden.

2. Verfahren nach Anspruch 1,
dadurch gekennzeichnet, daß
die kationische Stärke kationische Substituenten aufweist, die aus quaternären Ammoniumsalzgruppen bestehen.

3. Verfahren nach Anspruch 1 oder 2,
dadurch gekennzeichnet, daß
die wäßrige Lösung von kationischer Stärke, Wasserstoffperoxid und Halogenverbindung auf einem pH-Wert unter 7 gehalten wird.

4. Verfahren nach Anspruch 3,
dadurch gekennzeichnet, daß
der pH-Wert im Bereich von 4 bis 6 gehalten wird.

5. Verfahren nach einem der Ansprüche 1 bis 4,
dadurch gekennzeichnet, daß
die wäßrige Lösung auf einer Temperatur im Bereich von 20 bis 60 °C gehalten wird.

6. Verfahren nach einem der Ansprüche 1 bis 5,
dadurch gekennzeichnet, daß
das Molverhältnis Wasserstoffperoxid/Brom im Bereich von 1 bis 200 liegt.

7. Verfahren nach einem der Ansprüche 1 bis 5,
dadurch gekennzeichnet, daß
das Molverhältnis Wasserstoffperoxid/Bromwasserstoffsäure im Bereich von 0,5 bis 100 liegt.

8. Verfahren nach einem der Ansprüche 1 bis 7,
dadurch gekennzeichnet, daß
das Molverhältnis von Wasserstoffperoxid zu Anhydroglucoseeinheiten der als Ausgangsmaterial eingesetzten kationischen Stärke im Bereich von 0,1 bis 10 liegt.

9. Verfahren nach einem der Ansprüche 1 bis 8,
dadurch gekennzeichnet, daß
die wäßrige Lösung nach der Oxidation der kationischen Stärke einer Behandlung unterzogen wird, um die kationische Stärke mit Carboxygruppen in Form eines festen Niederschlags zu isolieren.

10. Verfahren nach Anspruch 9,
dadurch gekennzeichnet, daß
der wäßrigen Lösung ein Alkohol zugesetzt wird, um die kationische Stärke mit Carboxygruppen auszufällen.

11. Verfahren nach Anspruch 10,
dadurch gekennzeichnet, däß
der Alkohol unter Methanol und Ethanol ausgewählt ist.

12. Amphotere kationische Stärke mit Carboxygruppen,
dadurch gekennzeichnet, daß
ihre Carboxygruppen aus der selektiven Oxidation der Halbacetal-Endgruppen einer kationischen Stärke und ggf. der Halbacetal-Endgruppen der durch saure Hydrolyse dieser kationischen Stärke gebildeten Produkte herrühren.

## Claims

1. Process for the oxidation of a cationic starch containing terminal reducing groups, characterized in that the said cationic starch is reacted, in aqueous solution, with hydrogen peroxide and a halogen-containing compound chosen from amongst hydrobromic acid and bromine, so as to oxidize selectively the terminal reducing groups of the cationic starch and, where appropriate, the reducing groups of its products of acid hydrolysis, to give carboxyl groups.

2. Process according to Claim 1, characterized in that the cationic starch possesses cationic substituents consisting of quaternary ammonium salt groups.

3. Process according to Claim 1 or 2, characterized in that the said aqueous solution of cationic starch, hydrogen peroxide and halogen-containing compound is kept at a pH below 7.

4. Process according to Claim 3, characterized in that the said pH is kept at between 4 and 6.

5. Process according to one of Claims 1 to 4, characterized in that the aqueous solution is kept at a temperature of between 20 and 60°C.

6. Process according to any one of Claims 1 to 5, characterized in that the molar ratio of hydrogen peroxide/bromine is between 1 and 200.

7. Process according to one of Claims 1 to 5, characterized in that the molar ratio of hydrogen peroxide/hydrobromic acid is between 0.5 and 100.

8. Process according to one of Claims 1 to 7, characterized in that the molar ratio of hydrogen peroxide to the anhydroglucose units of the cationic starch starting material is between 0.1 and 10.

9. Process according to one of Claims 1 to 8, characterized in that, after the oxidation of the cationic starch, the aqueous solution is treated so as to isolate the carboxylic and cationic starch in the form of a solid precipitate.

10. Process according to Claim 9, characterized in that an alcohol is added to the aqueous solution to precipitate the carboxylic and cationic starch.

11. Process according to Claim 10, characterized in that the alcohol is chosen from amongst methanol and ethanol.

12. Amphoteric carboxylic and cationic starch characterized in that its carboxylic groups originate from the selective oxidation of the terminal hemiacetal groups of a cationic starch and possibly of the terminal hemiacetal groups of the products of acid hydrolysis of this cationic starch.
